# EUROPEAN PATENT APPLICATION

(11) **EP 4 589 320 A2**
(43) Date of publication of application: **23.07.2025**
(21) Application number: 25180219.5
(22) Date of filing: 26.04.2022
(51) Int. Cl.: G01R 33/34

(54) **INTERVENTIONAL LOCALIZATION GUIDE AND METHOD FOR MRI GUIDED PELVIC INTERVENTIONS**

(30) Priority: 26.04.2021 US 202163180013 P
(62) Divisional of application: 22723332.7
(71) Applicant: Promaxo, Inc., Oakland, CA 94607 (US)
(72) Inventor: KUMAR, Dinesh, Roseville, 95678 (US); NARAYANAN, Ram, San Jose, 95118 (US); NACEV, Aleksander, San Francisco, 94114 (US); HELLMAN, Eva, San Francisco, 94110 (US); SADINSKI, Meredith, Alameda, 94501 (US); ATHIVEERA RAMA PANDIAN, Sabareish, San Francisco, 94110 (US)
(74) Representative: Mathys & Squire

(57) **Abstract**

Interventional localization guides and methods for MRI guided pelvic interventions are disclosed. The interventional localization guides can include a stereotactic perineum positioning device having integrated MR receive coil array and fiducial receive array. The interventional localization guide can also include a physical template for guiding a surgical device, such as a biopsy needle. In various instances, the MRI guided pelvic interventions including co-registering biopsy locations on third party MRI scans.

## Description

### CROSS-REFERENCE TO RELATED APPLICATION

The present application claims the benefit of priority under 35 U.S.C. §119(e) to U.S. Provisional Application No. 63/180,013, entitled "INTERVENTIONAL LOCALIZATION GUIDE AND METHOD FOR MRI GUIDED PELVIC INTERVENTIONS", filed on April 26, 2021, which is incorporated by reference herein in its entirety.

### FIELD

The present disclosure is related to medical imaging, such as magnetic resonance imaging (MRI) techniques, for example, and/or various medical interventions, such as biopsy procedures, for example.

### BACKGROUND

MRI or other imaging modalities often use fiducials to demark the location of a patient's anatomy and/or location of an interventional device. However, these devices are often standalone and not incorporated into the magnetic resonance (MR) receive coil (RX coil). In addition, these devices typically are designed for the guidance of particular body parts. For example, current guide systems include stereotactic frames used for cranial interventions.

### SUMMARY

In one general aspect, the present disclosure provides a stereotactic perineum positioning device for magnetic resonance (MR) imaging. The stereotactic perineum positioning device comprises a frame, a patient receive coil rigidly mounted to the frame, and a fiducial array rigidly mounted to the frame. The fiducial array comprises three distinct MR-visible fiducials and a fiducial receive coil wrapped around the three distinct MR-visible fiducials.

In another aspect, the present disclosure provides a method, comprising acquiring, by a processor, a T2 scan acquired by an magnetic resonance imaging (MRI) system, wherein the T2 scan comprises a positioning device and MR-visible fiducials. The method further comprises localizing, by the processor, the MR-visible fiducials in the T2 scan, acquiring, by the processor, a third party MR image, and co-registering the MR-visible fiducials in the T2 scan with the third party MR image.

### BRIEF DESCRIPTION OF THE DRAWINGS

The novel features of the various aspects are set forth with particularity in the appended claims. The described aspects, however, both as to organization and methods of operation, may be best understood by reference to the following description, taken in conjunction with the accompanying drawings.
FIG. 1 is a perspective view of an MRI scanner, according to various aspects of the present disclosure.
FIG. 2 is an exploded, perspective view of the MRI scanner of FIG. 1, in which the permanent magnet assembly and the gradient coil sets within the housing are exposed, according to various aspects of the present disclosure.
FIG. 3 is an elevation view of the MRI scanner of FIG. 1, according to various aspects of the present disclosure.
FIG. 4 is an elevation view of the MRI scanner of FIG. 1, according to various aspects of the present disclosure.
FIG. 5 is a perspective view of the permanent magnet assembly of the MRI scanner of FIG. 1, according to various aspects of the present disclosure.
FIG. 6 is an elevation view of the gradient coil set and the permanent magnet assembly of the MRI system shown in FIG. 1, according to various aspects of the present disclosure.
FIG. 7 illustrates exemplary positioning of a patient for imaging by a single-sided MRI scanner for certain surgical procedures and interventions, according to various aspects of the present disclosure.
FIG. 8 is a control schematic for a single-sided MRI system, according to various aspects of the present disclosure.
FIG. 9 shows a magnetic resonance imaging system including a single-sided scanner or magnet cart, according to various aspects of the present disclosure.
FIG. 10 shows a patient in a lithotomy position for an imaging operation with the single-sided scanner of FIG. 9, according to various aspects of the present disclosure.
FIG. 11 depicts a perspective view of a stereotactic perineum positioning device for magnetic resonance (MR) imaging including a frame, an MR receive coil, and a fiducial arrangement, according to various aspects of the present disclosure.
FIG. 12 depicts another perspective view of the stereotactic perineum positioning device of FIG. 11, according to various aspects of the present disclosure.
FIG. 13 depicts a simplified view of the stereotactic perineum positioning device of FIG. 11, according to various aspects of the present disclosure.
FIG. 14 depicts positioning of the stereotactic perineum positioning device of FIG. 11 relative to a patient, according to various aspects of the present disclosure.
FIG. 15 depicts another view of positioning of the stereotactic perineum positioning device of FIG. 11 relative to a patient, according to various aspects of the present disclosure.
FIG. 16 depicts another view of positioning of the stereotactic perineum positioning device of FIG. 11 relative to a patient, according to various aspects of the present disclosure.
FIG. 17 depicts positioning of the stereotactic perineum positioning device of FIG. 11 relative to an MRI system, according to various aspects of the present disclosure.
FIG. 18 depicts positioning of the stereotactic perineum positioning device of FIG. 11 relative to a patient and an MRI system, according to various aspects of the present disclosure.
FIG. 19 depicts a view as seen through an access bore of the MRI system of FIG. 18, according to various aspects of the present disclosure.
FIG. 20 depicts positioning of the stereotactic perineum positioning device of FIG. 11 relative to a patient and an MRI system, according to various aspects of the present disclosure.
FIG. 21 depicts a close-up elevation view of the fiducial arrangement and physical template, according to various aspects of the present disclosure.
FIG. 22 shows the fiducial arrangement of FIG. 21 localized in an MR scan, according to various aspects of the present disclosure.
FIG. 23 is a flowchart depicting a clinical workflow for co-registering biopsy locations, according to various aspects of the present disclosure.

The exemplifications set out herein illustrate certain aspects of the invention, in one form, and such exemplifications are not to be construed as limiting the scope of the invention in any manner.

### DETAILED DESCRIPTION

The following international patent applications are incorporated by reference herein in their respective entireties:
- International Application No. PCT/US2020/018352, titled SYSTEMS AND METHODS FOR ULTRALOW FIELD RELAXATION DISPERSION, filed February 14, 2020, now International Publication No. WO2020/168233;
- International Application No. PCT/US2020/019530, titled SYSTEMS AND METHODS FOR PERFORMING MAGNETIC RESONANCE IMAGING, filed February 24, 2020, now International Publication No. WO2020/172673;
- International Application No. PCT/US2020/019524, titled PSEUDO-BIRDCAGE COIL WITH VARIABLE TUNING AND APPLICATIONS THEREOF, filed February 24, 2020, now International Publication No. WO2020/172672;
- International Application No. PCT/US2020/024776, titled SINGLE-SIDED FAST MRI GRADIENT FIELD COILS AND APPLICATIONS THEREOF, filed March 25, 2020, now International Publication No. WO2020/198395;
- International Application No. PCT/US2020/024778, titled SYSTEMS AND METHODS FOR VOLUMETRIC ACQUISITION IN A SINGLE-SIDED MRI SYSTEM, filed March 25, 2020, now International Publication No. WO2020/198396;
- International Application No. PCT/US2020/039667, title SYSTEMS AND METHODS FOR IMAGE RECONSTRUCTIONS IN MAGNETIC RESONANCE IMAGING, filed June 25, 2020, now International Publication No. WO2020/264194;
- International Application No. PCT/US2021/014628, titled MRI-GUIDED ROBOTIC SYSTEMS AND METHODS FOR BIOPSY, filed January 22, 2021;
- International Application No. PCT/US2021/018834, titled RADIO FREQUENCY RECEPTION COIL NETWORKS FOR SINGLE-SIDED MAGNETIC RESONANCE IMAGING, filed February 19, 2021; and
- International Patent Application PCT/US2021/21461, titled PULSE SEQUENCES AND FREQUENCY SWEEP PULSES FOR SINGLE-SIDED MAGNETIC RESONANCE IMAGING, filed March 9, 2021;
- International Patent Application PCT/US2021/21464, titled PHASE ENCODING WITH FREQUENCY SWEEP PULSES FOR MAGNETIC RESONANCE IMAGING IN INHOMOGENEOUS MAGNETIC FIELDS, filed March 9, 2021.

U.S. Patent Application Publication No. 2018/0356480, titled UNILATERAL MAGNETIC RESONANCE IMAGING SYSTEM WITH APERTURE FOR INTERVENTIONS AND METHODOLOGIES FOR OPERATING SAME, published December 13, 2018, is incorporated by reference herein in its entirety.

Before explaining various aspects of an magnetic resonance imaging (MRI) component, system, and method in detail, it should be noted that the illustrative examples are not limited in application or use to the details of construction and arrangement of parts illustrated in the accompanying drawings and description. The illustrative examples may be implemented or incorporated in other aspects, variations, and modifications, and may be practiced or carried out in various ways. Further, unless otherwise indicated, the terms and expressions employed herein have been chosen for the purpose of describing the illustrative examples for the convenience of the reader and are not for the purpose of limitation thereof. Also, it will be appreciated that one or more of the following-described aspects, expressions of aspects, and/or examples, can be combined with any one or more of the other following-described aspects, expressions of aspects, and/or examples.

In accordance with various aspects, an MRI system is provided that can include a unique imaging region that can be offset from the face of a magnet. Such offset and single-sided MRI systems are less restrictive as compared to traditional MRI scanners. In addition, this form factor can have a built-in or inherent magnetic field gradient that creates a range of magnetic field values over the region of interest. In other words, the inherent magnetic field can be inhomogeneous. The inhomogeneity of the magnetic field strength in the region of interest for the single-sided MRI system can be more than 200 parts per million (ppm). For example, the inhomogeneity of the magnetic field strength in the region of interest for the single-sided MRI system can between 200 ppm and 200,000 ppm. In various aspects of the present disclosure, the inhomogeneity in the region of interest can be greater than 1,000 ppm and can be greater than 10,000 ppm. In one instance, the inhomogeneity in the region of interest can be 81,000 ppm.

The inherent magnetic field gradient can be generated by a permanent magnet within the MRI scanner. The magnetic field strength in the region of interest for the single-sided MRI system can be less than 1 Tesla (T), for example. For example, the magnetic field strength in the region of interest for the single-sided MRI system can be less than 0.5 T. In other instances, the magnetic field strength can be greater than 1 T and may be 1.5 T, for example. This system can operate at a lower magnetic field strength as compared to typical MRI systems allowing for a relaxation on the RX coil design constraints and/or allowing for additional mechanisms, like robotics, for example, to be used with the MRI scanner. Exemplary MRI-guided robotic systems are further described in International Application No. PCT/US2021/014628, titled MRI-GUIDED ROBOTIC SYSTEMS AND METHODS FOR BIOPSY, filed January 22, 2021, for example.

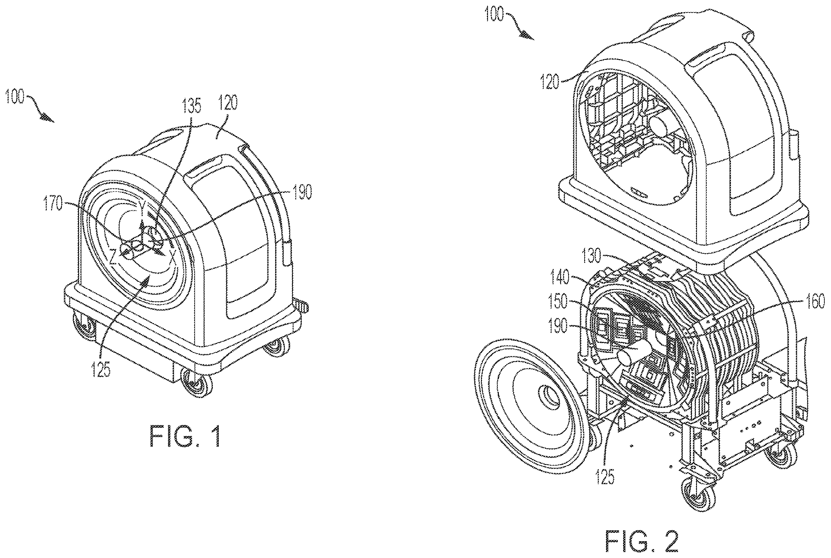

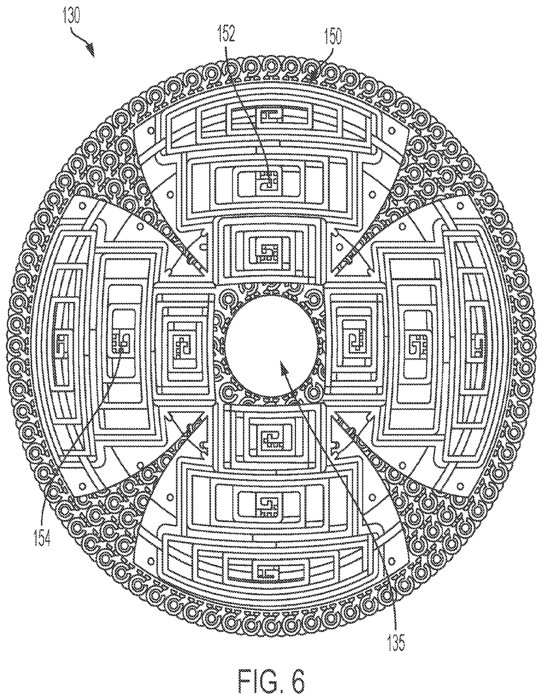

FIGS. 1-6 depict an MRI scanner 100 and components thereof. As shown in FIGS. 1 and 2, the MRI scanner 100 includes a housing 120 having a face or front surface 125, which is concave and recessed. In other aspects, the face of the housing 120 can be flat and planar. The front surface 125 can face the object being imaged by the MRI scanner. As shown in FIGS. 1 and 2, the housing 120 includes a permanent magnet assembly 130, an RF transmission coil (TX) 140, a gradient coil set 150, an electromagnet 160, and a RF reception coil (RX) 170. In other instances, the housing 120 may not include the electromagnet 160. Moreover, in certain instances, the RF reception coil 170 and the RF transmission coil 140 can be incorporated into a combined Tx/Rx coil array.

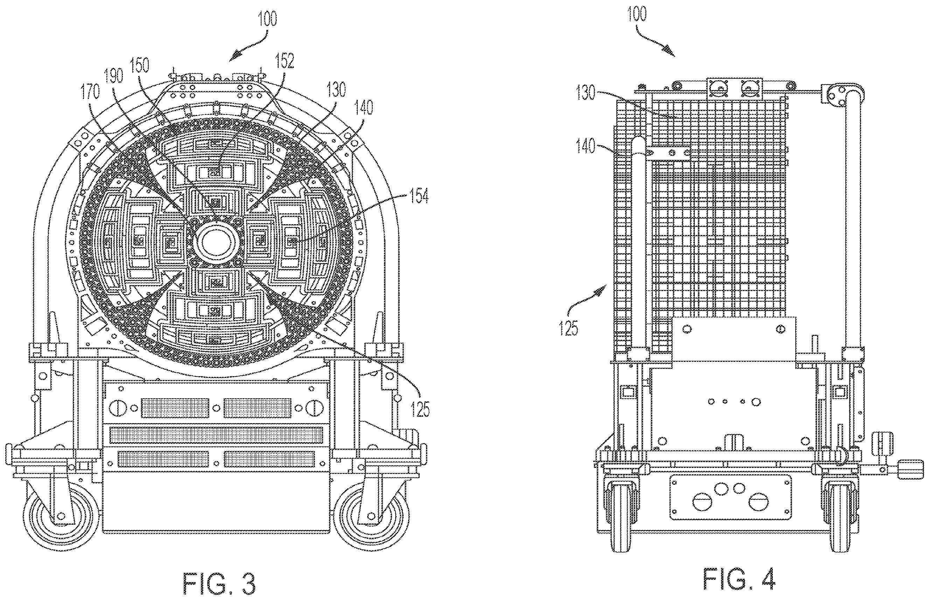

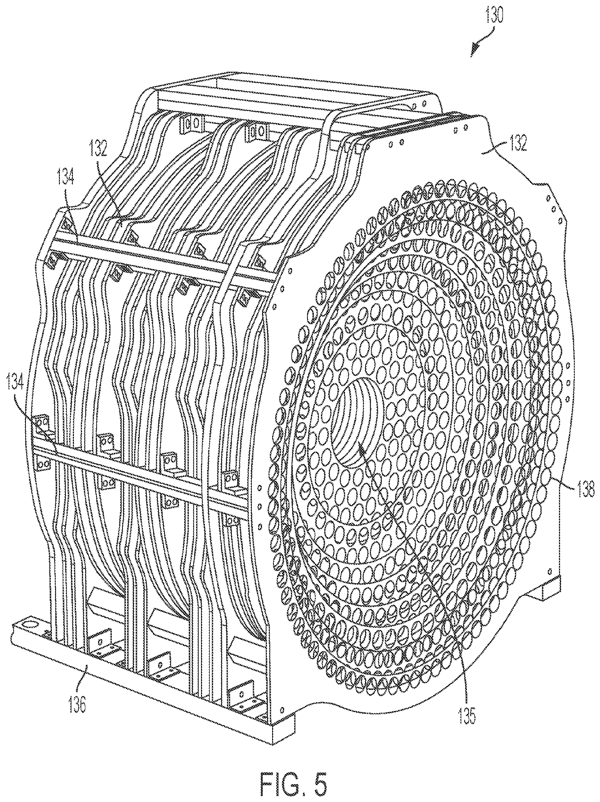

Referring primarily to FIGS. 3-5, the permanent magnet assembly 130 includes an array of magnets. The array of magnets forming the permanent magnet assembly 130 are configured to cover the front surface 125, or patient-facing surface, of the MRI scanner 100 (see FIG. 3) and are shown as horizontal bars in FIG. 4. The permanent magnet assembly 130 includes a plurality of cylindrical permanent magnets in a parallel configuration. Referring primarily to FIG. 5, the permanent magnet assembly 130 comprises parallel plates 132 that are held together by brackets 134. The system can be attached to the housing 120 of the MRI scanner 100 at a bracket 136. There can be a plurality of holes 138 in the parallel plates 132. The permanent magnet assembly 130 can include any suitable magnetic materials, including but not limited to rare-earth based magnetic materials, such as for example, Neodymium-based magnetic materials, for example.

The permanent magnet assembly 130 defines an access aperture or bore 135, which can provide access to the patient through the housing 120 from the opposite side of the housing 120. In other aspects of the present disclosure, the array of permanent magnets forming a permanent magnet assembly in the housing 120 may be bore-less and define an uninterrupted or contiguous arrangement of permanent magnets without a bore defined therethrough. In still other instances, the array of permanent magnets in the housing 120 may form more than one bore/access aperture therethrough.

In accordance with various aspects of the present disclosure, the permanent magnet assembly 130 provides a magnetic field B0 in a region of interest 190 that is along the Z axis, shown in FIG. 1. The Z axis is perpendicular to the permanent magnet assembly 130. Stated differently, the Z axis extends from a center of the permanent magnet assembly 130 and defines a direction of the magnetic field B0 away from the face of the permanent magnet assembly 130. The Z axis can define the primary magnetic field B0 direction. The primary magnetic field B0 can decrease along the Z axis, i.e. an inherent gradient, farther from the face of the permanent magnet assembly 130 and in the direction indicated with the arrow in FIG. 1.

In one aspect, the inhomogeneity of the magnetic field in the region of interest 190 for the permanent magnet assembly 130 can be approximately 81,000 ppm. In another aspect, the inhomogeneity of the magnetic field strength in the region of interest 190 for the permanent magnet assembly 130 can be between 200 ppm to 200,000 ppm and can be greater than 1,000 ppm in certain instances, and greater than 10,000 ppm in various instances.

In one aspect, the magnetic field strength of the permanent magnet assembly 130 can be less than 1 T. In another aspect, the magnetic field strength of the permanent magnet assembly 130 can be less than 0.5 T. In other instances, the magnetic field strength of the permanent magnet assembly 130 can be greater than 1 T and may be 1.5 T, for example. Referring primarily to FIG. 1., the Y axis extends up and down from the Z axis and the X axis extends to the left and right from the Z axis. The X axis, the Y axis, and the Z axis are all orthogonal to one another and the positive direction of each axis is indicated by the corresponding arrow in FIG. 1.

The RF transmission coils 140 are configured to transmit RF waveforms and associated electromagnetic fields. The RF pulses from the RF transmission coils 140 are configured to rotate the magnetization produced by the permanent magnet 130 by generating an effective magnetic field, referred to as B1, that is orthogonal to the direction of the permanent magnetic field (e.g. an orthogonal plane).

Referring primarily to FIG. 3, the gradient coil set 150 includes two sets of gradient coils 152, 154. The sets of gradient coils 152, 154 are positioned on the face or front surface 125 of the permanent magnet assembly 130 intermediate the permanent magnet assembly 130 and the region of interest 190. Each set of gradient coils 152, 154 includes a coil portion on opposing sides of the bore 135. Referring to the axes in FIG. 1, the gradient coil set 154 may be the gradient coil set corresponding to the X axis, for example, and the gradient coil set 152 may be the gradient coil set corresponding to the Y axis, for example. The gradient coils 152, 154 enable encoding along the X axis and Y axis, as further described herein.

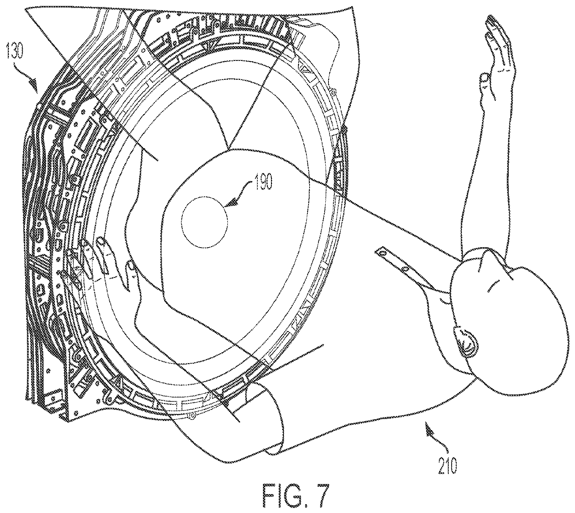

In accordance with various aspects, using the MRI scanner 100 illustrated in FIGS. 1-6, a patient can be positioned in any number of different positions depending on the type of anatomical scan. FIG. 7 shows an example where the pelvis is scanned with the MRI scanner 100. To perform the scan, a patient 210 can be laid on a surface in a lithotomy position. As illustrated in FIG. 7, for the pelvic scan, the patient 210 can be positioned to have their back resting on a table and legs raised up to be resting against the top of the scanner 100. The pelvic region can be positioned directly in front of the permanent magnet assembly 130 and the bore 135 and the region of interest 190 is in the pelvic region of the patient 210.

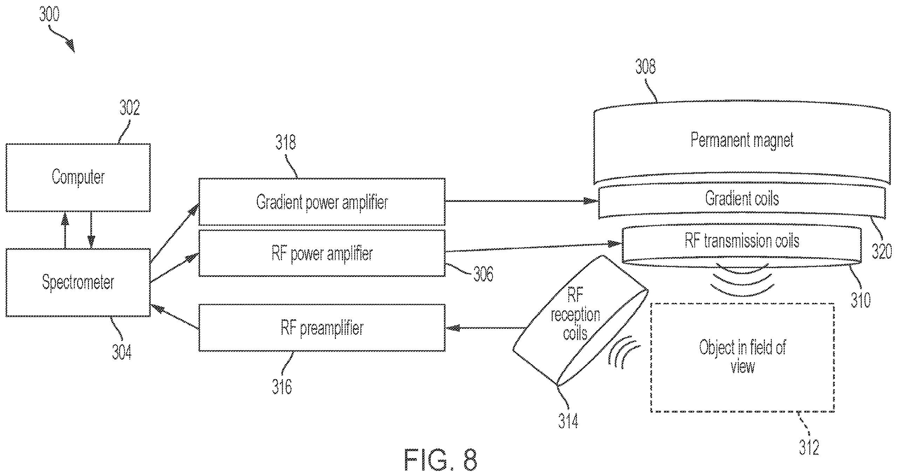

Referring now to FIG. 8, a control schematic for a single-sided MRI system 300 is shown. The single-sided MRI scanner 100 and/or components thereof (FIGS. 1-6) can be incorporated into the MRI system 300 in various aspects of the present disclosure. For example, the imaging system 300 includes a permanent magnet assembly 308, which can be similar to the permanent magnet assembly 130 (see FIGS. 2-5) in various instances. The imaging system 300 also includes RF transmission coils 310, which can be similar to the RF transmission coil 140 (see FIG. 3), for example. Moreover, the imaging system 300 includes RF reception coils 314, which can be similar to the RF reception coils 170 (see FIG. 3), for example. In various aspects, the RF transmission coils 310 and/or the RF reception coils can also be positioned in the housing of an MRI scanner and, in certain instances, the RF transmission coils 310 and the RF reception coils 314 can be combined into integrated Tx/Rx coils. The system 300 also includes gradient coils 320, which are configured to generate gradient fields to facilitate imaging of the object in the field of view 312.

The single-sided MRI system 300 also includes a computer 302, which is in signal communication with a spectrometer 304, and is configured to send and receive signals between the computer 302 and the spectrometer 304.

The main magnetic field B0 generated by the permanent magnet 308 extends away from the permanent magnet 308 and away from the RF transmission coils 310 into the field of view 312. The field of view 312 contains an object that is being imaged by the MRI system 300.

During the imaging process, the main magnetic field B0 extends into the field of view 312. The direction of the effective magnetic field B1 changes in response to the RF pulses and associated electromagnetic fields from the RF transmission coils 310. For example, the RF transmission coils 310 are configured to selectively transmit RF signals or pulses to an object in the field of view, e.g. tissue. These RF pulses alter the effective magnetic field experienced by the spins in the sample (e.g. patient tissue). When the RF pulses are on, the effective field experienced by spins on resonance is solely the RF pulse, effectively canceling the static B0 field. The RF pulses can be chirp or frequency sweep pulses, for example, as further described herein.

Moreover, when the object in the field of view 312 is excited with RF pulses from the RF transmission coils 310, the precession of the object results in an induced electric current, or MR current, which is detected by the RF reception coils 314. The RF reception coils 314 can send the excitation data to an RF preamplifier 316. The RF preamplifier 316 can boost or amplify the excitation data signals and send them to the spectrometer 304. The spectrometer 304 can send the excitation data to the computer 302 for storage, analysis, and image construction. The computer 302 can combine multiple stored excitation data signals to create an image, for example.

From the spectrometer 304, signals can also be relayed to the RF transmission coils 310 via an RF power amplifier 306, and to the gradient coils 320 via a gradient power amplifier 318. The RF power amplifier 306 amplifies the signal and sends it to RF transmission coils 310. The gradient power amplifier 318 amplifies the gradient coil signal and sends it to the gradient coils 320.

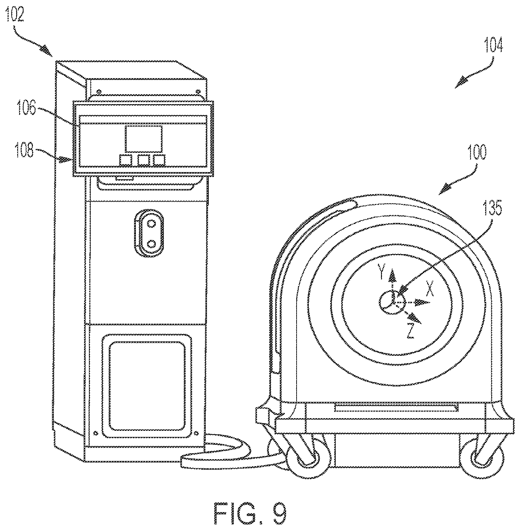

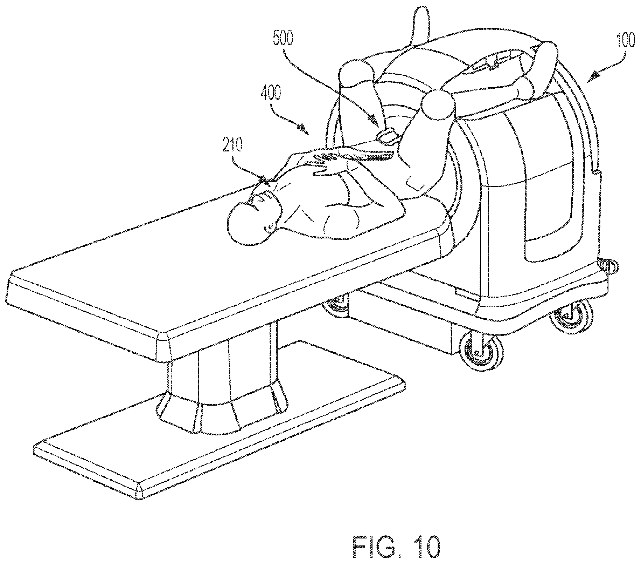

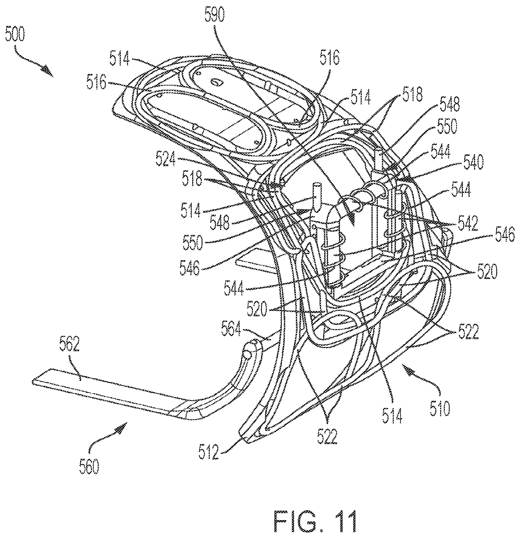

An interventional localization guide or device can allow for the integration of a fiducial tracker, i.e. fiducial arrangement 540 (FIG. 11), on top of or in combination with an interventional device (e.g. a template for biopsy guidance) and can also be integrated with a MR receive coil 510 (FIG. 11), for example. The MR receive coil 510 (FIG 11) and fiducial tracker 540 (FIG. 11) can be used with a single-sided MRI scanner 100 for prostate interventions in certain instances. A single-sided, low-field MRI system 104 is shown in FIG. 9, in which an auxiliary cart 102 can house the electrical and electronic components, such as a computer, display 106, user interface 108, programmable logic controller, power distribution unit, and amplifiers, for example. A magnet cart, i.e. single-sided MRI scanner 100, can house a magnet, gradient coils, and a transmission coil and can attach to the receive coil. The single-sided, low-field MRI scanner 100 can be utilized with a lithotomy scanning position 400 in which the patient's legs are positioned around the MRI scanner 100, as depicted in FIG. 10.

The region of interest can be offset from the face of the magnet cart along a Z axis defined by the permanent magnet housed therein. The magnetic field strength in the region of interest for the single-sided MRI system can be less than 1 Tesla (T), for example. For example, the magnetic field strength in the region of interest for the single-sided MRI system can be less than 0.5 T. In other instances, the magnetic field strength can be greater than 1 T and may be 1.5 T, for example.

Various single-sided MRI systems are further described in U.S. Patent Application Publication No. 2018/0356480, titled UNILATERAL MAGNETIC RESONANCE IMAGING SYSTEM WITH APERTURE FOR INTERVENTIONS AND METHODOLOGIES FOR OPERATING SAME, published December 13, 2018, and various additional references that have been incorporated by reference herein.

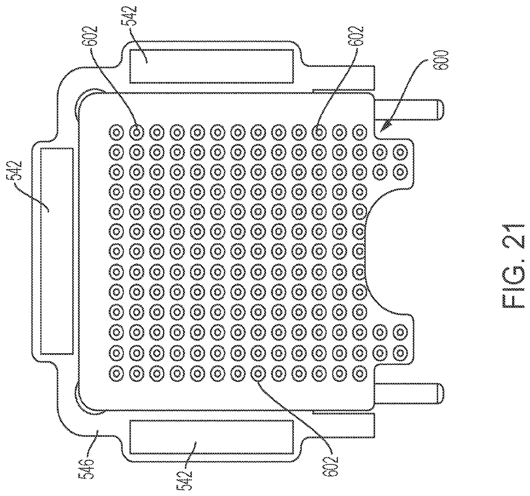

The integrated MR receive coil 510 and fiducial tracker 540 can act like a stereotactic perineum positioning device, i.e. interventional localization guide 500, for imaging of the pelvis, for example. The device creates a common coordinate system for registering MR-acquired images to a physical template 600 (FIG. 21) or structure adjacent to the patient's anatomy allowing for guidance of interventional devices, including but not limited to prostate biopsy guns, cryotherapy needles, or brachytherapy needles relative to the patient's anatomy and, in certain instances, with the benefit of intraoperative MR imaging. Stated differently, the stereotactic perineum positioning device including the MR receive coil 510 and fiducial tracker system 540 with fiducials 542 and a physical template 600 (FIG. 21) can form an intraoperative interventional localization guide 500 for synchronous prostrate imaging and biopsy.

The stereotactic structure can include two coil arrangements: (A) a fiducial coil 544, i.e. fiducial receive coil / fiducial RF coil / fiducial solenoid, which incorporates multiple separate fiducials 542 and (B) an MR receive coil 510, i.e. main radio frequency (RF) receive coil / patient coil / patient-wearable receive coil, that surrounds at least a portion of the patient's pelvis. With respect to the fiducial receive coil 544, three separate fiducials 542 are each wound with a connected solenoid surrounding a rectangular space into which a biopsy template or guidance structure can be rigidly attached. The fiducials 542 can be made from mineral oil or constructed from any MR-visible substance.

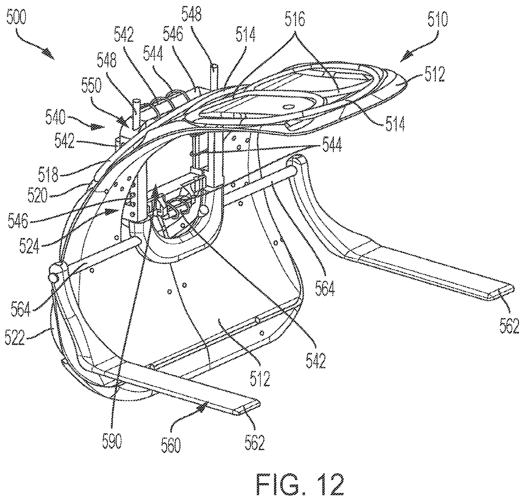

The MR receive coil 510 is positioned within a housing or enclosure 512, which houses the different coils 514, 516, 518, 520, and 522 that make up the MR receive coil 510. In the example embodiment shown in FIGS. 11 and 12, the MR receive coil 510 comprises five coils 514, 516, 518, 520, and 522. The coils 514, 516, 518, 520, and 522 are butterfly coils comprising a pair of lobes. The first coil 514 forms a first lobe or loop at an upper portion of the array and a second lobe or loop in a middle portion of the array. The first loop of the first coil 514 surrounds the second coil 516. The second loop of the first coil 514 surrounds a through hole 524 in the enclosure 512. The second coil 516 is located above the through hole 524. The third coil 518 extends around the upper half of the through hole 524. The fourth coil 520 extends around the lower half of the through hole 524. Ends of the loops of the third and fourth coil 518, 520 overlap at a vertical centerline through the through hole 524. The first coil 514 also overlaps/underlaps a portion of the second coil 516, the third coil 518, and the fourth coil 520. The fifth coil 522 is positioned along a lower portion of the enclosure 512 below the through hole 524. All of the coils 514, 516 518, 520, and 522 overlap each other in areas so that at least a portion of each coil sits on top of a portion of one other coil in order to form an overlapping array.

The enclosure 512 also defines a curve. In other embodiments, the enclosure 512 and coils therein can define a different radius of curvature or multiple different radii of curvature. A different number of coils could be included in alternative MR receive coil and/or the coils could comprise different geometries and/or sizes, for example.

The fiducial RF coil 544 wrapped around the fiducials 542 can be one large solenoid. In other instances, it can be of any RF design created in such a way to pick up a signal from the fiducials 542. The fiducial RF coil 544 could also be incorporated into the main RF receive coil 510 in certain instances. The fiducial RF coil 544 is mounted to a support structure that can be designed with a crossbar 564 and generally horizontal supports 562 configured to be inserted below the patient's back when in a supine/lithotomy position, for example, so that it is rigidly registered to the patient's perineum. The fiducial RF coil 544 can be translated in the y-direction (up and down) and can be positioned in such a way that it is centered at the perineum above the rectum and encompassing the y-direction expanse of the prostate.

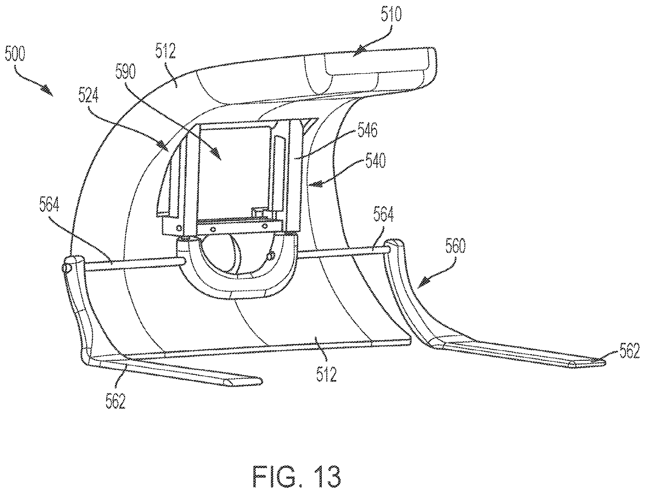

The main RF receive coil 510 surrounds the patient's pelvis to optimize the signal acquisition from the prostate region. This coil 510 also contains a large access port 524 designed so that the fiducial RF coil 544 can be mated to the main RF receive coil 510. This allows for the two coils 510, 544 to be co-located and rigidly attached to the patient during the image acquisition. In various instances, the components can be mated by simply placing one on top of and/or adjacent to the other (see, e.g., FIGS. 11-13). For example, the two coils 510, 544 can be sandwiched with each other and pressed against the anatomy. In other instances, geometrical features such as a ridge/groove is configured to lock them into place while allowing for a single degree of freedom of movement (e.g., up and down in the vertical direction).

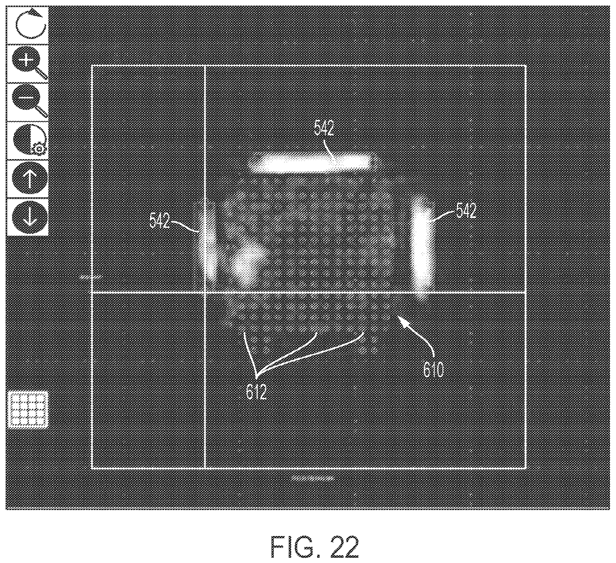

Referring to FIGS. 11-13, an interventional localization guide 500 including a frame 512, an arrangement of MR-visible fiducials 542 and a fiducial receive coil 544, and a main RF receive coil 510 are shown. The interventional localization guide 500 is designed to have a through hole 590 that can allow a clinician access through the interventional localization guide 500. The main RF receive coil 510 is comprised of multiple receive coil elements 514, 516 518, 520, and 522 that are mounted to the frame. The MR-visible fiducials 542 can be seen on an MRI, see FIG. 22. The fiducial receive coil 544 is wrapped around the MR-visible fiducials 542. A goalpost-style holder 560 is configured to position the two coils (MR receive coil 510 and fiducial receive coil 544) relative to each other. For example, MR-invisible material (e.g. ceramics) can allow the two structures 546, 560 (FIGS. 11-13) to be attached together. In certain instances, ceramic rods 548 can be received within cylindrical bores 550 in the two structures 546, 560. For example, the fiducial coil frame 546 can include cylindrical bores 550 extending along a vertical direction, and the ceramic rods 548 can be inserted within these bores 550. The height of the fiducial coil frame 546 along the ceramic rods 548 can be adjusted by the application of a small plastic set screw that extends into the fiducial coil frame 546 and applies pressure to the ceramic rod 548. In various instances, the ceramic rods 548 can also be adjustably positioned in cylindrical bores 550 in the goalpost-style holder 560. The goalpost-style holder 560 can utilize ceramic rods 564, which can allow for rotation about the rods 564 (e.g. a rotation about the x-axis). Set screws can similarly secure the ceramic rods 564 relative to the goalpost-style holder 560. In such instances, the two structures 546, 560 can be secured together in different geometries/shapes to accommodate patients of different sizes and shapes.

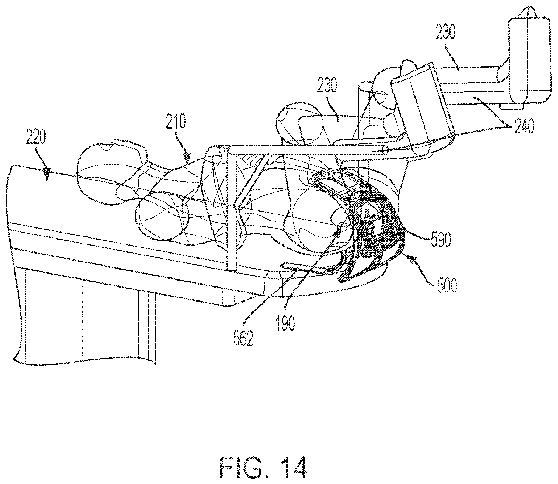

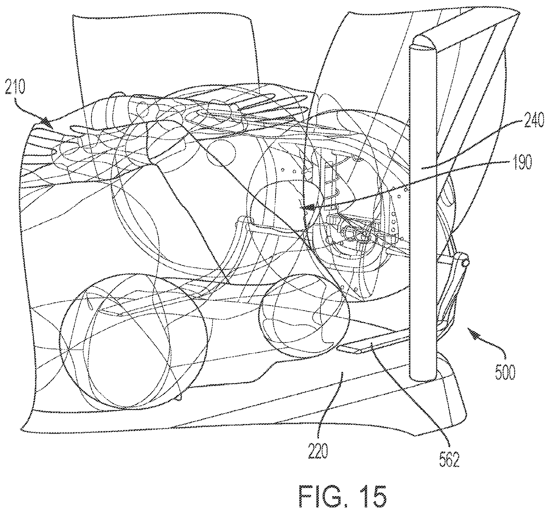

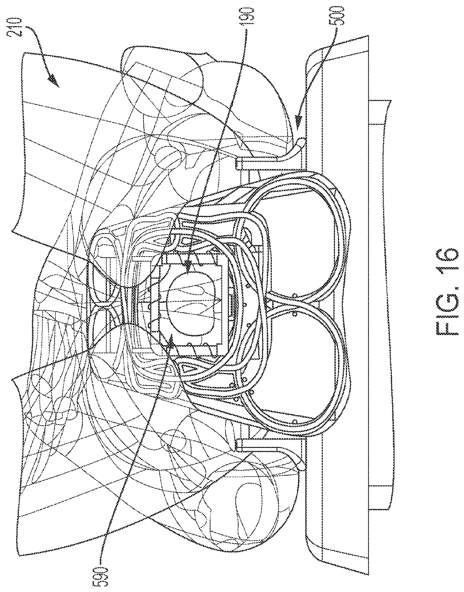

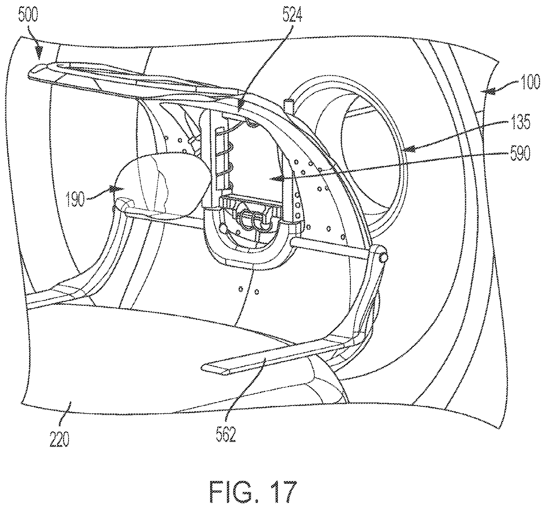

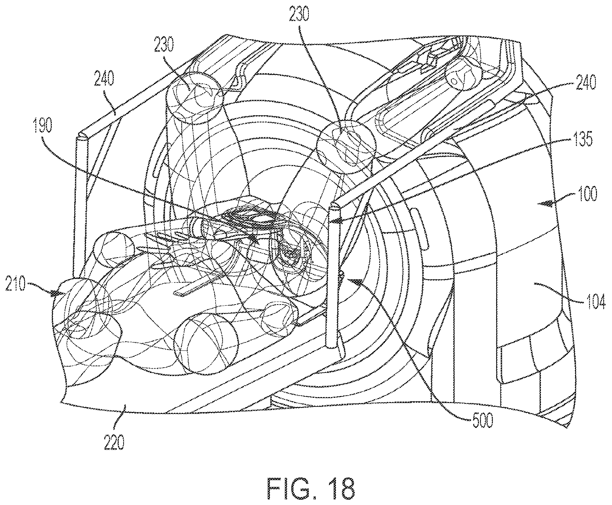

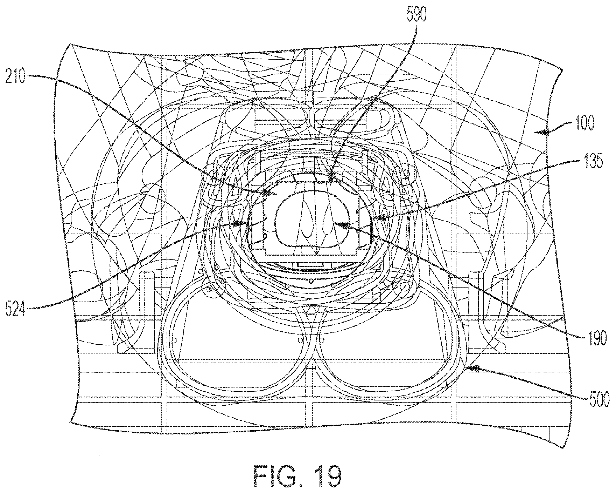

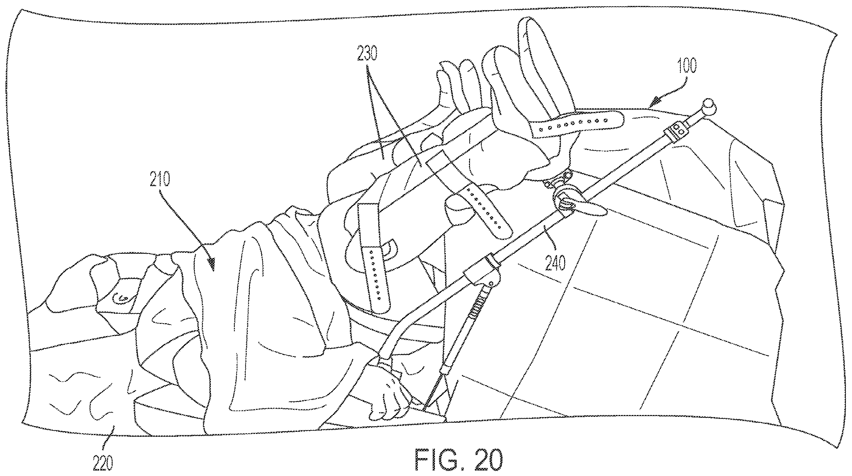

The placement of a main RF receive coil 510 (i.e. patient coil) and the fiducial receive coils 544 relative to a patient 210 and an MRI system 104 are shown in FIGS. 14-20. FIG. 14 shows a patient 210 positioned in a lithotomy position on a table 220 with each leg 230 in a brace 240 attached to the table 220. The horizontal supports 562 of the interventional localization guide 500 can be slid under the patient's 210 back holding the interventional localization guide 500 close to the patient's 210 prostate. FIGS. 15 and 16 show different close up views of the interventional localization guide 500 being positioned against the patient 210. The region of interest 190 represents the patient's 210 prostate in FIGS. 11-19. FIG. 16 provides a view showing a through hole 590 in the interventional localization guide 500, which allows a clinician access through the interventional localization guide 500 to the region of interest 190. FIG. 17 shows the positioning of the interventional localization guide 500 relative to MRI scanner 100. The access bore 135 of the MRI system is aligned with the through hole 590 of the interventional localization guide 500. This alignment provides access for a clinician to the region of interest 190. For example, the clinician can access the region of interest 190 by going through the access bore 135 of the MRI scanner 100 and then through the through hole 590 of the interventional localization guide 500 to reach the region of interest 190. FIG. 18 shows how the patient 210, interventional localization guide 500, and the MRI scanner 100 are positioned. The patient 210 is in the lithotomy position and the braces 240 position the patient's legs 230 around the MRI scanner 100 of the MRI system 104. FIG. 19 shows the access for a clinician to the region of interest 190 through the access bore 135 and through hole 590. For example, a clinician could use a surgical device to extend through the access bore 135 to the region of interest. It is also noted that a clinician could use a surgical device to reach around the MRI scanner 100 (not using the access bore 135) to reach the region of interest.

In at least one aspect of the present disclosure, the MRI biopsy localization system includes two coils 510, 544 designed to surround a patient's lower abdomen and provide guidance using MR-visible fiducials 544 for the purpose of a prostate biopsy. The patient receive coil network is positioned as close as possible to the patient's prostate to maximize signal acquisition from the region. As described in various additional references described herein, see, e.g. International Application No. PCT/US2021/018834, titled RADIO FREQUENCY RECEPTION COIL NETWORKS FOR SINGLE-SIDED MAGNETIC RESONANCE IMAGING, filed February 19, 2021, due to the direction of the main magnetic field used to align the spin of the protons, the main RF receive coil 510 (RX coil) needs to be sensitive to a direction perpendicular to the main field direction. Referring still to FIGS. 14-20, that direction is perpendicular to the access bore 135 of the magnet. The fiducial receive coil 544 has the same requirement. However, since the sample can be fully enclosed by a coil, a solenoid can be wrapped around each MR visible fiducial element 542. Referring primarily again to FIGS. 11 and 12, the fiducial arrangement 540 includes a fiducial receive coil 544 wrapped around four fiducials 542 (three fiducials seen in the front view (FIG. 11) and a small additional fiducial 542 is positioned near the bottom, backside of the structure 546 (FIG. 12)). In other instances, the fiducial arrangement 540 includes three fiducials 542. In various instances, three separate fiducials 542 are needed to localize the template to a plane within the MR imaging space. Fewer or more fiducials 542 are possible in certain instances provided there are at least three points to register. Therefore, in many instances, three separate fiducials 542 are preferred. The fiducial arrangement 540 also has a space for the insertion of a biopsy template 600 (FIG. 21) showing access ports 602. The biopsy template 600 can be registered into the MR frame of reference, shown as a virtual template 610 showing the access ports 612 (FIG. 22), and used to identify locations from which to acquire a biopsy. FIG. 22 shows the biopsy template 600 and access ports 602 shown in the MR frame of reference as the virtual template 610 and access ports 612. The patient 210 and fiducial coil 544 are co-located in such a way to position the fiducials 542 orthogonal to the MR access aperture 135 while the patient receive coil 510 interlocks with the fiducial receive coil 544.

In various instances, the interventional localization guide 500 can be utilized with a robotic system and a robotic arm can extend through an access bore 135 and/or around the perimeter of the magnet cart 100 to the patient 210 and/or region of interest 190, as further described in International Application No. PCT/US2021/014628, titled MRI-GUIDED ROBOTIC SYSTEMS AND METHODS FOR BIOPSY, filed January 22, 2021, which has been incorporated by reference herein in its entirety.

In certain instances, the fiducial arrangement 540 does not need to have separate receive coils and instead the fiducials 542 can be imaged using the patient wearable receive coil.

In certain instances, there could be more or less than three fiducials 542 as long as three distinct regions can be identified.

In various instances, both receive coils 510, 544 can be combined into one structure as long as the resultant structure is rigidly attached and located to the patient to ensure a static patient frame of reference.

In one or more instances, an endorectal coil could be used.

In certain instances, the fiducials 542 can be constructed of MR-visible materials that are not at the hydrogen resonant frequency potentially necessitating a differently tuned receive coil array for localizing the fiducials 542.

The template might be sized differently (e.g. larger or smaller) for different interventional therapies.

In various instances, there may not be a template and the coordinate system might be shared with a robotic interventional device to perform the interventions.

In certain instances, MR-visible fiducials 542 may not be needed and instead a small receive coil can be used in conjunction with an applied gradient or RF signal produced by the system to localize the small pickup coils in three-dimensional space. By utilizing a spatially varying electromagnetic or RF transmission field, the voltage induced into the small receive coil will be dependent upon the signal strength sent through the gradient or RF coils as well as the position in three-dimensional space. By mapping the field a priori, the spatial location of the pick-up loop and therefore the template can be localized in space.

In various instances, an acquisition method for an MRI image can allow for the acquisition of an MRI image of a rigidly attached fiducial 542 to extract the patient's frame of reference. This frame of reference can then allow a clinician to select and target desired foci within the body. The following methodology for a clinical workflow allows for the co-registration of externally-acquired MR images, recently-acquired MR images, and an attached physical template 600 to a common frame of reference. In various instances, the attached physical template 600 can be the stereotactic perineum positioning device shown in FIGS. 11-22.

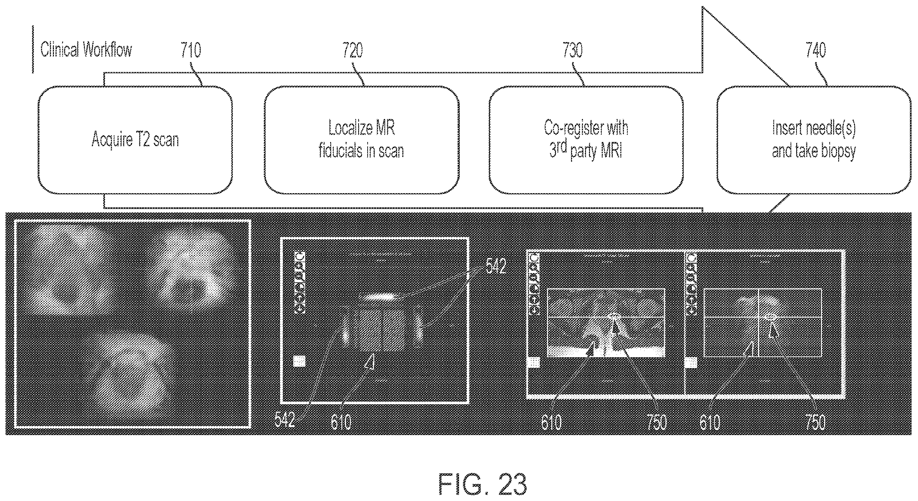

In one aspect of the present disclosure, referring now to FIG. 23, a T2 scan is acquired (710) with the physical template 600, e.g. a stereotactic perineum positioning device having an RF receive coil and fiducial receive coil array/registration, as further described herein. The MR fiducials 542 are localized within the scan and registered in a frame of reference (720). The T2 scan is then registered to a third party MRI scan using anatomical landmarks (730). These registrations create a common frame of reference between all images and the physical world. From the third party scan or from the acquired T2 scan, a region of interest 750 is selected and the three-dimensional coordinate of the interventional site is calculated. From this coordinate, a depth and desired access port 602 on the physical template 600 is determined. In the embodiment of FIG. 23, a biopsy needle is inserted into the physical template 600 and guided to the appropriate depth (740).

The interventional procedure can be any number of prostate or pelvic interventional procedures, such as cryotherapy, brachytherapy, etc.

In various instances, multiple third party images can be co-registered to the acquired scans.

In certain instances, an image can also be acquired during or after the intervention to confirm the location of the targeted intervention.

In at least one aspect of the current disclosure, an endorectal coil could be used.

In various instances, the template might be larger or smaller for different interventional therapies. In still other instances, there may not be a template as the coordinate system might be shared with a robotic interventional device to perform the interventions.

In certain instances, MR-visible fiducials may not be needed. Instead a small receive coil can be used in conjunction with an applied gradient or RF signal produced by the system to localize the small pickup coils in three-dimensional space.

### EXAMPLES

Various aspects of the subject matter described herein are set out in the following numbered examples.

Example 1. A stereotactic perineum positioning device for magnetic resonance (MR) imaging. The stereotactic perineum positioning device comprises a frame, a patient receive coil rigidly mounted to the frame, and a fiducial array rigidly mounted to the frame. The fiducial array comprises three distinct MR-visible fiducials and a fiducial receive coil wrapped around the three distinct MR-visible fiducials.

Example 2. The stereotactic perineum positioning device of Example 1, wherein the patient receive coil comprises a plurality of coils symmetrically oriented about a vertical centerline of the frame.

Example 3. The stereotactic perineum positioning device of any one of Examples 1 or 2, wherein the frame comprises an access bore.

Example 4. The stereotactic perineum positioning device of Example 3, wherein the plurality of coils are positioned around the access bore.

Example 5. The stereotactic perineum positioning device of any one of Examples 3, or 4, wherein the fiducial array is at least partially positioned in the access bore.

Example 6. The stereotactic perineum positioning device of any one of Examples 1, 2, 3, 4, or 5, wherein the frame comprises a contoured side profile configured to curve around a patient in a lithotomy position.

Example 7. The stereotactic perineum positioning device of any one of Examples 1, 2, 3, 4, 5, or 6, further comprising a support structure mounted to the frame and configured to extend under the patient and to stabilize the frame relative to a patient's perineum.

Example 8. The stereotactic perineum positioning device of any one of Examples 1, 2, 3, 4, 5, 6, or 7, further comprising a physical template.

Example 9. The stereotactic perineum positioning device of Example 8, wherein the fiducial array comprises a U-shaped holder configured to support the three distinct MR-visible fiducials, and wherein the physical template is configured to slide into the U-shaped holder.

Example 10. The stereotactic perineum positioning device of any one of Examples 8 or 9, wherein the physical template comprises an array of needle-receiving through-holes.

Example 11. A method, comprising acquiring, by a processor, a T2 scan acquired by an magnetic resonance imaging (MRI) system, wherein the T2 scan comprises a positioning device and MR-visible fiducials. The method further comprises localizing, by the processor, the MR-visible fiducials in the T2 scan, acquiring, by the processor, a third party MR image, and co- registering the MR-visible fiducials in the T2 scan with the third party MR image.

Example 12. The method of Example 11, further comprising calculating, by the processor, a three-dimensional coordinate of an anatomical target.

Example 13. The method of any one of Examples 11 or 12, further comprising guiding, by the processor, a biopsy needle through the positioning device to the three-dimensional coordinate.

Example 14. The method of Example 13, wherein a robot arm is configured to position the biopsy needle at the three-dimensional coordinate.

Example 15. The method of any one of Examples 13 or 14, further comprising obtaining, by the MRI system, intraoperative MR images, as the biopsy needle is guided through the positioning device.

Example 16. The method of Example 15, further comprising localizing, by the processor, the MR-visible fiducials in the intraoperative MR images, and co-registering the MR- visible fiducials in the intraoperative MR images with the third party MR image.

Example 17. The method of any one of Examples 11, 12, 13, 14, 15, or 16, wherein the method comprises a prostate interventional procedure.

Example 18. The method of any one of Examples 11, 12 ,13, 14, 15, 16, or 17, further comprising imaging a region of interest with the MRI system, and wherein the MRI system comprises a single-sided, low-field MRI scanner.

Example 19. The method of any one of Examples 11, 12, 13, 14, 15, 16, 17, or 18, further comprising positioning a stereotactic perineum positioning device adjacent to a patient's pelvic region, wherein the stereotactic perineum positioning device comprises a frame, a radio frequency receive coil rigidly mounted to the frame, and a fiducial arrangement rigidly mounted to the frame, wherein the fiducial arrangement comprises MR-visible fiducials and a fiducial receive coil wrapped around the MR-visible fiducials.

While several forms have been illustrated and described, it is not the intention of Applicant to restrict or limit the scope of the appended claims to such detail. Numerous modifications, variations, changes, substitutions, combinations, and equivalents to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms. The appended claims are intended to cover all such modifications, variations, changes, substitutions, modifications, and equivalents.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, and/or examples can be implemented, individually and/or collectively, by a wide range of hardware, software, firmware, or virtually any combination thereof. Those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as one or more program products in a variety of forms, and that an illustrative form of the subject matter described herein applies regardless of the particular type of signal bearing medium used to actually carry out the distribution.

Instructions used to program logic to perform various disclosed aspects can be stored within a memory in the system, such as dynamic random access memory (DRAM), cache, flash memory, or other storage. Furthermore, the instructions can be distributed via a network or by way of other computer readable media. Thus a machine-readable medium may include any mechanism for storing or transmitting information in a form readable by a machine (e.g., a computer), but is not limited to, floppy diskettes, optical disks, compact disc, read-only memory (CD-ROMs), and magneto-optical disks, read-only memory (ROMs), random access memory (RAM), erasable programmable read-only memory (EPROM), electrically erasable programmable read-only memory (EEPROM), magnetic or optical cards, flash memory, or a tangible, machine-readable storage used in the transmission of information over the Internet via electrical, optical, acoustical or other forms of propagated signals (e.g., carrier waves, infrared signals, digital signals, etc.). Accordingly, the non-transitory computer-readable medium includes any type of tangible machine-readable medium suitable for storing or transmitting electronic instructions or information in a form readable by a machine (e.g., a computer).

As used in any aspect herein, the term "control circuit" may refer to, for example, hardwired circuitry, programmable circuitry (e.g., a computer processor including one or more individual instruction processing cores, processing unit, processor, microcontroller, microcontroller unit, controller, digital signal processor (DSP), programmable logic device (PLD), programmable logic array (PLA), or field programmable gate array (FPGA)), state machine circuitry, firmware that stores instructions executed by programmable circuitry, and any combination thereof. The control circuit may, collectively or individually, be embodied as circuitry that forms part of a larger system, for example, an integrated circuit (IC), an application-specific integrated circuit (ASIC), a system on-chip (SoC), desktop computers, laptop computers, tablet computers, servers, smart phones, etc. Accordingly, as used herein "control circuit" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

As used in any aspect herein, the term "logic" may refer to an app, software, firmware and/or circuitry configured to perform any of the aforementioned operations. Software may be embodied as a software package, code, instructions, instruction sets and/or data recorded on non-transitory computer readable storage medium. Firmware may be embodied as code, instructions or instruction sets and/or data that are hard-coded (e.g., nonvolatile) in memory devices.

As used in any aspect herein, the terms "component," "system," "module" and the like can refer to a computer-related entity, either hardware, a combination of hardware and software, software, or software in execution.

As used in any aspect herein, an "algorithm" refers to a self-consistent sequence of steps leading to a desired result, where a "step" refers to a manipulation of physical quantities and/or logic states which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities and/or states.

Unless specifically stated otherwise as apparent from the foregoing disclosure, it is appreciated that, throughout the foregoing disclosure, discussions using terms such as "processing," "computing," "calculating," "determining," "displaying," or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

One or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

The terms "proximal" and "distal" are used herein with reference to a clinician manipulating the handle portion, or housing, of a surgical instrument. The term "proximal" refers to the portion closest to the clinician and/or to the robotic arm and the term "distal" refers to the portion located away from the clinician and/or from the robotic arm. It will be further appreciated that, for convenience and clarity, spatial terms such as "vertical", "horizontal", "up", and "down" may be used herein with respect to the drawings. However, robotic surgical tools are used in many orientations and positions, and these terms are not intended to be limiting and/or absolute.

Those skilled in the art will recognize that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flow diagrams are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "an exemplification," "one exemplification," and the like means that a particular feature, structure, or characteristic described in connection with the aspect is included in at least one aspect. Thus, appearances of the phrases "in one aspect," "in an aspect," "in an exemplification," and "in one exemplification" in various places throughout the specification are not necessarily all referring to the same aspect. Furthermore, the particular features, structures or characteristics may be combined in any suitable manner in one or more aspects.

Any patent application, patent, non-patent publication, or other disclosure material referred to in this specification and/or listed in any Application Data Sheet is incorporated by reference herein, to the extent that the incorporated materials is not inconsistent herewith. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

Embodiments of the disclosure are set out in the following numbered clauses:
1. A stereotactic perineum positioning device for magnetic resonance (MR) imaging, the stereotactic perineum positioning device comprising:
   a frame;
   a patient receive coil rigidly mounted to the frame; and
   a fiducial array rigidly mounted to the frame, wherein the fiducial array comprises three distinct MR-visible fiducials and a fiducial receive coil wrapped around the three distinct MR- visible fiducials.
2. The stereotactic perineum positioning device of Clause 1, wherein the patient receive coil comprises a plurality of coils symmetrically oriented about a vertical centerline of the frame.
3. The stereotactic perineum positioning device of Clause 2, wherein the frame comprises an access bore.
4. The stereotactic perineum positioning device of Clause 3, wherein the plurality of coils are positioned around the access bore.
5. The stereotactic perineum positioning device of Clause 4, wherein the fiducial array is at least partially positioned in the access bore.
6. The stereotactic perineum positioning device of Clause 1, wherein the frame comprises a contoured side profile configured to curve around a patient in a lithotomy position.
7. The stereotactic perineum positioning device of Clause 6, further comprising a support structure mounted to the frame and configured to extend under the patient and to stabilize the frame relative to a patient's perineum.
8. The stereotactic perineum positioning device of Clause 1, further comprising a physical template.
9. The stereotactic perineum positioning device of Clause 8, wherein the fiducial array comprises a U-shaped holder configured to support the three distinct MR-visible fiducials, and wherein the physical template is configured to slide into the U-shaped holder.
10. The stereotactic perineum positioning device of Clause 9, wherein the physical template comprises an array of needle-receiving through-holes.
11. A method, comprising:
   acquiring, by a processor, a T2 scan acquired by an magnetic resonance imaging (MRI) system, wherein the T2 scan comprises a positioning device and MR-visible fiducials;
   localizing, by the processor, the MR-visible fiducials in the T2 scan;
   acquiring, by the processor, a third party MR image; and
   co-registering the MR-visible fiducials in the T2 scan with the third party MR image.
12. The method of Clause 11, further comprising calculating, by the processor, a three-dimensional coordinate of an anatomical target.
13. The method of Clause 12, further comprising guiding, by the processor, a biopsy needle through the positioning device to the three-dimensional coordinate.
14. The method of Clause 13, wherein a robot arm is configured to position the biopsy needle at the three-dimensional coordinate.
15. The method of Clause 14, further comprising obtaining, by the MRI system, intraoperative MR images, as the biopsy needle is guided through the positioning device.
16. The method of Clause 15, further comprising:
   localizing, by the processor, the MR-visible fiducials in the intraoperative MR images; and
   co-registering the MR-visible fiducials in the intraoperative MR images with the third party MR image.
17. The method of Clause 11, wherein the method comprises a prostate interventional procedure.
18. The method of Clause 11, further comprising imaging a region of interest with the MRI system, and wherein the MRI system comprises a single-sided, low-field MRI scanner.
19. The method of Clause 11, further comprising positioning a stereotactic perineum positioning device adjacent to a patient's pelvic region, wherein the stereotactic perineum positioning device comprises a frame, a radio frequency receive coil rigidly mounted to the frame, and a fiducial arrangement rigidly mounted to the frame, wherein the fiducial arrangement comprises MR-visible fiducials and a fiducial receive coil wrapped around the MR- visible fiducials.

## Claims

1. A method, comprising:
acquiring, by a processor, a T2 scan acquired by a magnetic resonance imaging (MRI) system, wherein the T2 scan comprises a positioning device and MR-visible fiducials;
localizing, by the processor, the MR-visible fiducials in the T2 scan;
acquiring, by the processor, a third-party MR image; and
co-registering the MR-visible fiducials in the T2 scan with the third-party MR image.

2. The method of Claim 1, further comprising calculating, by the processor, a three-dimensional coordinate of an anatomical target.

3. The method of Claim 2, further comprising guiding, by the processor, a biopsy needle through the positioning device to the three-dimensional coordinate.

4. The method of Claim 3, wherein a robot arm is configured to position the biopsy needle at the three-dimensional coordinate.

5. The method of Claim 4, further comprising obtaining, by the MRI system, intraoperative MR images, as the biopsy needle is guided through the positioning device.

6. The method of Claim 5, further comprising:
localizing, by the processor, the MR-visible fiducials in the intraoperative MR images; and
co-registering the MR-visible fiducials in the intraoperative MR images with the third-party MR image.

7. The method of Claim 1, wherein the method comprises a prostate interventional procedure.

8. The method of Claim 1, further comprising imaging a region of interest with the MRI system, and wherein the MRI system comprises a single-sided, low-field MRI scanner.

9. The method of Claim 1, further comprising positioning a stereotactic perineum positioning device adjacent to a patient's pelvic region, wherein the stereotactic perineum positioning device comprises a frame, a radio frequency receive coil rigidly mounted to the frame, and a fiducial arrangement rigidly mounted to the frame, wherein the fiducial arrangement comprises MR-visible fiducials and a fiducial receive coil wrapped around the MR-visible fiducials.
